# EUROPEAN PATENT APPLICATION

(11) **EP 3 605 085 A1**
(43) Date of publication of application: **05.02.2020**
(21) Application number: 18776204.2
(22) Date of filing: 20.02.2018
(51) Int. Cl.: G01N 33/48, G01N 33/483

(54) **INFORMATION PROCESSING DEVICE, INFORMATION PROCESSING METHOD, AND CELL ANALYSIS SYSTEM**

(30) Priority: 31.03.2017 JP 2017071280
(71) Applicant: Sony Corporation, Tokyo 108-0075 (JP)
(72) Inventor: ABE, Tomoteru, Tokyo 108-0075 (JP)
(74) Representative: D Young & Co LLP
(86) International application number: PCT/JP2018/005918
(87) International publication number: WO 2018/180007

(57) **Abstract**

The present technology provides a technology capable of analyzing efficacy of a drug in a subject before administration or at an early stage after administration in cancer immunotherapy with an immune checkpoint inhibitor.

For this purpose, the present technology provides an information processing device and the like at least including an analyzing unit which analyzes a result of measurement over time of oxygen consumption rates of different types of immune cells collected from a subject stored for each cell in sealable micro-wells and/or a change in pH around the immune cell, and an output unit which outputs a prediction result of efficacy of an anticancer drug in the subject on the basis of a result of the analysis.

## Description

### TECHNICAL FIELD

The present technology relates to an information processing device, an information processing method, and a cell analyzing system. More specifically, this relates to an information processing device, an information processing method, and a cell analyzing method which may be a cell analyzing method capable of analyzing efficacy of a drug in a subject before administration or at an early stage after the administration in cancer immunotherapy by an immune checkpoint inhibitor.

### BACKGROUND ART

In recent years, researches regarding a correlation between cancer cells and immune cells of patients progress, and a mechanism by which the cancer cells escape from recognition by the immune cells and elimination by cytotoxic activity is clarified at a molecular level. Then, on the basis of this understanding, a new drug referred to as an immune checkpoint inhibitor which induces shrinkage or elimination of the cancer cells by the immune cells of the patient by inhibiting a means by which the cancer cells avoid immune cell attack has been developed.

As the immune checkpoint inhibitor, nivolumab (OPDIVO (registered trademark)), a PD-1 inhibitor first approved in Japan, had significantly high treatment results to some types of cancers in which conventional chemotherapy fails, and the number of applications drastically increased. However, on the other hand, it becomes clear that there are cases with high efficacy and cases with almost no efficacy even in similar cancer cases, which causes serious problems in terms of medical economy as well as side effects and treatment results.

From them, development of a method capable of determining the efficacy in individual patients of the drug as accurately as possible before administration is desired.

Unlike conventional chemotherapy to kill the cancer cells directly by chemical substances, the immune checkpoint inhibitor uses a drug which exerts an effect while acting on a relationship between the immune cells of the patient and the cancer cells. Therefore, an object thereof is to change not sensitivity of the cancer cells to the chemical substances but a state of immune cells.

As a result, in order to predict the efficacy thereof, it is necessary to analyze a change in state of the immune cells of the patient caused by the drug or natures of the immune cells of the patient themselves.

This is indicated by, for example, data and the like indicating that a shrinkage effect of tumor caused by administration of a PD-1 inhibitor is highly correlated with a change in oxygen consumption of immune cells of a mouse in which the tumor is transplanted in an experiment of the mouse to which the tumor is transplanted in Non-Patent Document 1.

However, types of immune cells are very diverse, and researches regarding classification are also in progress. Furthermore, a function of each individual immune cell is also complicated, and it is predicted to be extremely difficult to predict efficacy based on understanding of a mechanism such as a cell and a change of the cell in response to a certain drug and stimulation which leads to cancer shrinkage or absence of effect.

Furthermore, due to the extremely large number of classes of immune cells and a problem of heterogeneity of cells within the same class, combinations of reactions of the respective cells to each drug stimulation and the like and changes in nature might have an enormous number of patterns and complexity, so that it is extremely difficult to find a specific combination with human eyes.

### CITATION LIST

### NON-PATENT DOCUMENT

Non-patent document 1: Proc Natl Acad Sci U S A. 2017 Jan 31; 114(5): E761-E770.

### SUMMARY OF THE INVENTION

### PROBLEMS TO BE SOLVED BY THE INVENTION

Therefore, a principal object of the present technology is to provide a technology capable of analyzing efficacy of a drug in a subject before administration or at an early stage after administration in cancer immunotherapy with an immune checkpoint inhibitor.

### SOLUTIONS TO PROBLEMS

The present technology first provides an information processing device at least including an analyzing unit which analyzes a result of measurement over time of oxygen consumption rates of different types of immune cells collected from a subject stored for each cell in sealable micro-wells and/or a change in pH around the immune cell, and an output unit which outputs a prediction result of efficacy of an anticancer drug in the subject on the basis of a result of the analysis.

In the information processing device according to the present technology, the measured result may be a result measured when the immune cell is stressed by drug stimulation and/or a change in external environment.

Furthermore, in the information processing device according to the present technology, the measured result may be a result measured by using an oxygen sensor and/or a pH sensor immobilized in at least a part of the micro-well. In this case, the micro-well may be formed by using a translucent material, and the measured result may be a result of measurement over time of a change of the oxygen sensor and/or a change of the pH sensor by using an optical means with respect to the micro-well. Furthermore, in this case, the analyzing unit may further analyze a decay time of phosphorescence or fluorescence obtained from the oxygen sensor and/or the pH sensor.

Moreover, in the information processing device according to the present technology, the analyzing unit may further perform profiling of energy metabolism of each immune cell on the basis of the measured result. In this case, the profiling may be performed by analyzing a subtype of the immune cell determined from analysis of a surface antigen of the immune cell and a combination of energy metabolism of the immune cell of each subtype.

In addition, in the information processing device according to the present technology, the immune cell may include any one selected from a group of CD8 positive T cells, CD4 positive helper T cells, and CD19 positive B cells.

Furthermore, in the information processing device according to the present technology, an oligonucleotide for mRNA capturing may be arranged in advance in at least a part of the micro-well. In this case, the oligonucleotide may be an identification sequence different for a part or all of the micro-wells.

Moreover, in the information processing device according to the present technology, the anticancer drug may be a molecular target drug. In this case, the molecular target drug may be a drug containing an anti-PD-1 antibody as an active ingredient.

Furthermore, the present technology also provides an information processing method at least performing an analyzing procedure of analyzing a result of measurement over time of oxygen consumption rates of different types of immune cells collected from a subject stored for each cell in sealable micro-wells and/or a change in pH around the immune cell, and an output procedure of outputting a prediction result of efficacy of an anticancer drug in the subject on the basis of a result of the analysis.

Moreover, the present technology also provides a cell analyzing system at least including micro-wells which sealably store different types of immune cells collected from a subject, a detecting device which detects an oxygen consumption rate of each of the immune cells and/or a change in pH around the immune cell, and an information processing device which analyzes the oxygen consumption rate of the immune cell and/or the change in pH around the immune cell and outputs a prediction result of efficacy of an anticancer drug in the subject on the basis of a result of the analysis.

### EFFECTS OF THE INVENTION

According to the present technology, it is possible to analyze efficacy of a drug in a subject before administration or at an early stage after administration in cancer immunotherapy with an immune checkpoint inhibitor. Note that, the effects herein described are not necessarily limited and may be any of the effects described in the present disclosure.

### BRIEF DESCRIPTION OF DRAWINGS

Fig. 1 is a flowchart illustrating an example of an information processing method according to the present technology.
Fig. 2 is a schematic diagram schematically illustrating a cell analyzing system 10 according to the present technology.

### MODE FOR CARRYING OUT THE INVENTION

Hereinafter, a preferred mode for carrying out the present technology is described with reference to the drawings. An embodiment hereinafter described describes an example of a representative embodiment of the present technology, and the scope of the present technology is not narrowed by this. Note that, the description is given in the following order.
1. Information processing device 1
   (1) Analyzing unit 11
   (2) Output unit 12
   (3) Other units
2. Information processing method
3. Cell analyzing system 10
   (1) Micro-well 101
   (2) Detecting Device 102
   (3) Information processing device 1
   (4) Others

### 1. Information processing device 1

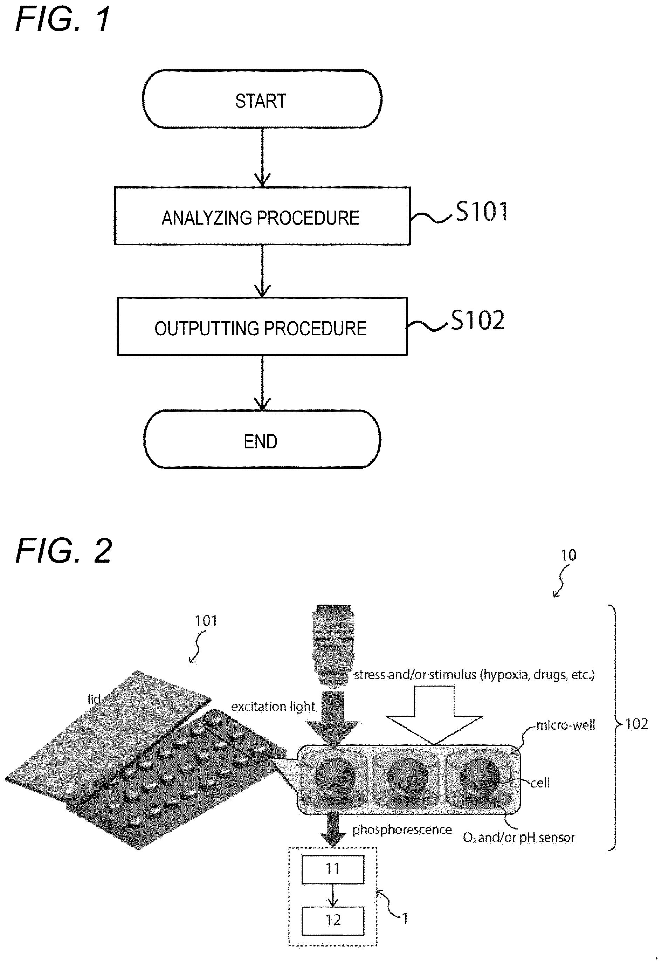

An information processing device 1 according to the present technology is at least provided with an analyzing unit 11 and an output unit 12. Furthermore, another unit may also be provided as needed. Fig. 2 is a schematic diagram schematically illustrating a cell analyzing system 10 according to the present technology in a part of which the information processing device 1 is illustrated. Hereinafter, each unit is described in detail.

### (1) Analyzing unit 11

The analyzing unit 11 is a unit which analyzes a result of measurement over time of oxygen consumption rates of different types of immune cells collected from a subject stored for each cell in sealable micro-wells and/or a change in pH around the immune cell.

A cell mainly synthesizes ATP by glucose metabolism by a glycolytic system and mitochondrial oxidative phosphorylation. It is known that in a case where the glycolytic system is dominant in a process of ATP synthesis, lactic acid which is a metabolic product thereof is released out of the cell, and the pH around the cell decreases by an effect thereof. Furthermore, in a case where the oxidative phosphorylation is dominant, oxygen is used as an electron acceptor, resulting in an increase in oxygen consumption by the cell.

Therefore, it is possible to discriminate an energy metabolism pattern of each cell by using the result of measurement over time of the oxygen consumption rate for each immune cell and the change in pH around the same in a state in which the immune cells are stored in the micro-wells for each cell.

In other words, with this arrangement, the present technology may analyze a dynamic change for each cell. As an analysis target, for example, in addition to the energy metabolism mentioned here, there may be secretion of cytokines, other phenotypes, gene expression, surface antigens and the like.

In the present technology, the measured result may be a result measured when the immune cell is stressed by drug stimulation and/or a change in external environment. Examples of the drugs include, for example, drugs including anticancer drugs, low-molecular substances, proteins which might be antigens, sugar chains and the like. The stress caused by the change in external environment includes, for example, hypoxic stress caused when an environment in which the immune cell is placed is put into a hypoxic state, stress caused by a change in temperature and the like.

With this arrangement, it is possible to continuously observe an immune cell response to these externally administered drugs and/or the stress due to the change in external environment and reflect the same in the analysis. Then, for example, it is possible to evaluate activity of the immune cell in an environment close to a hypoxic state in a microenvironment in cancer tissue. Furthermore, by analyzing the phenotype and dynamic change for each immune cell, it is possible to associate the immune cells, the stimulation, and the phenotypes which the immune cells express by the stimulation with one another.

Furthermore, in the present technology, integrated analysis such as profiling of the energy metabolism of each immune cell may also be performed on the basis of the measured result.

Specifically, for example, the profiling may be performed by analyzing a subtype of the immune cell determined by analysis of the surface antigen of the immune cell and a combination of energy metabolism of the immune cell of each subtype.

In addition to this, in the present technology, it is also possible to analyze by using various machine learning methods such as deep leaning to analyze in a multidimensional manner which phenotype is expressed by which immune cell by which stimulation, and a duration time and a pattern of intensity thereof, and determine whether or not an effect of the anticancer drug being a determination target is high or determine a type of disease while checking against many cases, clinical trial results, article data and the like.

In the present technology, a means of sealing the micro-well is not especially limited, and for example, there may be a means of sealing the micro-well and the like, by using a lid, mineral oil or the like. This makes it possible to measure the oxygen consumption rate of the cell and the change in pH around the same.

Furthermore, in the present technology, the measurement over time of the oxygen consumption rate for each immune cell and the change in pH around the same may be performed in the information processing device 1, but may also be performed using an external device. In this case, the information processing device 1 according to the present technology and the external device may be connected to each other via a network.

Although a measuring method is not especially limited, a method performed by using an oxygen sensor and/or a pH sensor immobilized in at least a part of the micro-well is preferable. In other words, it is preferable that the measured result is a result measured by using the oxygen sensor and/or the pH sensor immobilized in at least a part of the micro-well. At least a part of the micro-well may be a part of a bottom surface of the micro-well, a part of an inner side of the lid and the like, for example.

As the oxygen sensor, for example, platinum octaethylporphyrin (PtOEP) or the like is used, and as the pH sensor, for example, Eu(III)-CS370-DTPA-hydrazide dye or the like is used.

In the present technology, a material of the micro-well is not especially limited, but it is preferable to form the micro-well using a translucent material. With this arrangement, the measured result may be a result obtained by measuring a change of the oxygen sensor and/or a change of the pH sensor over time by using an optical means with respect to the micro-well.

One of meanings of measuring the change of the oxygen sensor and/or the change of the pH sensor over time by such a method is that, by utilizing characteristics that intensity and a decay time constant of emitted phosphorescence or fluorescence change depending on an oxygen concentration or the pH, it becomes possible to detect the lights in a state without noise by excitation light.

Therefore, in the present technology, it is preferable that the analyzing unit 11 further analyzes a decay time of the phosphorescence or fluorescence obtained from the oxygen sensor and/or the pH sensor. A method of detecting the phosphorescence or fluorescence is not especially limited; for example, as illustrated in Fig. 2, in a state in which the immune cell is stored in the micro-well arranged on an image sensor, the excitation light may be applied from above, and thereafter, the decay of the phosphorescence or fluorescence obtained may be detected by the image sensor on a lower part, and the like. By adopting this method, it becomes possible to analyze a large number of immune cells at once, and it becomes possible to analyze the clinically significant number of cells in a realistic time.

The immune cells are not especially limited, but they are preferably a group of selected cells the types and subtypes of which are identified by antibody staining of cell surface antigens and the like, and include an arbitrary group of immune cells by a selecting method thereof.

Specifically, for example, there may be CD8 positive T cells known to be activated by antigen presentation of cancer antigens and to exert cytotoxic activity, CD4 positive helper T cells which positively or negatively control their functions, furthermore, CD19 positive B cells which control tumor immunity by secretion of various cytokines, and the like. In other words, the immune cells preferably include any one selected from a group including the CD8 positive T cells, CD4 positive helper T cells, and CD19 positive B cells.

In the present technology, an oligonucleotide for mRNA capturing may be arranged in advance in at least a part of the micro-well. With this arrangement, it is possible to trap single cell-derived mRNA in situ by performing dissolving or permeation treatment on the immune cells by adding a solubilizing agent after filling of the immune cells. Thereafter, transcriptome analysis by single cell-derived mRNA sequencing becomes possible by recovering and amplifying this mRNA or cDNA synthesized with this as a template, and the like. Then, it is possible to analyze while associating the previously analyzed phenotypes and gene expression.

Furthermore, in the present technology, the oligonucleotide may be an identification sequence different for a part or all of the micro-wells. With this arrangement, it becomes possible to trap the single cell-derived mRNA having a different identification sequence in situ, which leads to efficiency of the analysis described above.

Note that, at least a part of the micro-well herein specifically includes a part of the bottom surface of the micro-well, a part of the inner side of the lid and the like, for example, as that described above.

In the present technology, the anticancer drug is not especially limited, but is preferably a molecular target drug. The molecular target drug is known to exert an antitumor effect mainly by acting on ability of the cancer cells to suppress or avoid antitumor activity of the immune cells as an action mechanism. For this reason, it is considered that whether or not the administered anticancer drug has efficacy depends on an immunological escape mechanism of the cancer cells to maintain their proliferation ability.

Therefore, for example, it has conventionally tried to determine efficacy of a drug containing an anti-PD-1 antibody as an active ingredient depending on whether or not the cancer cells of a patient express PD-L1 molecules. However, recent studies prove that patients cannot be sufficiently stratified with this method, and there is a need to develop a more accurate method.

However, on the other hand, in order to make this possible, it is tried to realize this by analyzing the number of immune cells of the patient and distribution of subtypes classified by the surface antigens with a flow cytometer, or by measuring secretory capacity of cytokines by a method such as ELISPOT. However, with these methods, it is no more than to watch a snapshot of the immune cell at a certain timing, so that information sufficient for determination cannot be obtained. Therefore, in order to realize a highly accurate inspection, it is necessary to analyze the dynamic change of immune cells placed in various environments.

On the other hand, it becomes possible to analyze the dynamic changes of the immune cells placed in various environments for each single cell as described above by using the information processing device 1 according to the present technology, the present technology is very useful in determining the efficacy of the molecular target drug.

In the present technology, the molecular target drug is not especially limited, but is preferably various cancer immunotherapy drugs, and specifically, for example, the drug containing the anti-PD-1 antibody as the active ingredient.

### (2) Output unit 12

The output unit 12 is a unit which outputs a prediction result of the efficacy of the anticancer drug in the subject on the basis of the result of the analysis.

The output is output to an external device (for example, a computer, a CPU and the like) and displayed on a display, a printer and the like provided in the device.

Furthermore, in the present technology, an output result may also be stored in a memory such as a hard disk. Note that the memory may be connected to the information processing device 1 according to the present technology via a network.

### (3) Other units

The information processing device 1 according to the present technology may be provided with, in addition to the analyzing unit 11 and the output unit 12 described above, other units such as a data accumulating unit which accumulates the result of the analysis as long as the effects of the present technology are not impaired. Note that, in the present technology, the data accumulating unit may be included in the analyzing unit 11 described above.

Note that, in the present technology, it is possible to store functions performed by respective units of the information processing device 1 according to the present technology in a personal computer and a hardware resource provided with a control unit including a CPU and the like and a recording medium (for example, a nonvolatile memory (for example, USB memory and the like), HDD, CD and the like) and the like as a program, and allow the same to function by the personal computer or the control unit.

As described above, by using the information processing device 1 according to the present technology, it becomes possible to analyze the oxygen consumption rate for each immune cell and the change in pH around the same, and the profiling of the energy metabolism becomes possible at a single cell level. Furthermore, the dynamic changes of various immune cells may be analyzed for each single cell. Then, static phenotypes such as the surface antigens and morphological characteristics, dynamic phenotypes by kinetic changes, and further, genotypes such as the gene expression and DNA sequence may be analyzed in an integrated manner.

In addition, by using the information processing device 1 according to the present technology, it becomes possible to analyze an immune status of the subject in detail, and it is possible to obtain detailed data for determining the efficacy of the drug in the subject before the administration or at an early stage after the administration in cancer immunotherapy with an immune checkpoint inhibitor.

### 2. Information processing method

Fig. 1 is a flowchart illustrating an example of an information processing method according to the present technology. The information processing method according to the present technology performs at least an analyzing procedure S101 and an output procedure S102. The method performed in the analyzing procedure S101 is the same as the method performed by the analyzing unit 11 described above, and the method performed in the output procedure S102 is the same as the method performed by the output unit 12 described above, so that the description is herein omitted.

The information processing method according to the present technology may also be provided with, in addition to the analyzing procedure S101 and the output procedure S102 described above, other procedures such as a data accumulating procedure for accumulating the result of the analysis as long as the effects of the present technology are not impaired. Note that, in the present technology, the data accumulating procedure may also be included in the analyzing procedure S101 described above.

### 3. Cell analyzing system 10

Fig. 2 is a schematic diagram schematically illustrating the cell analyzing system 10 according to the present technology. The cell analyzing system 10 according to the present technology is at least provided with micro-wells 101 which sealably store different types of immune cells collected from a subject, a detecting device 102 which detects an oxygen consumption rate of each of the immune cells and/or a change in pH around the immune cell, and an information processing device 1 which analyzes the oxygen consumption rate of the immune cell and/or the change in pH around the immune cell and outputs a prediction result of efficacy of an anticancer drug in the subject on the basis of the result of the analysis. Furthermore, other devices and the like may also be provided as needed. Hereinafter, each unit is described in detail.

### (1) Micro-well 101

The micro-well 101 is not especially limited as long as this may sealably store different types of immune cells collected from the subject. Details thereof are the same as the contents described in "1. Information processing device 1", so that the description thereof is herein omitted.

### (2) Detecting Device 102

The detecting device 102 is not especially limited as long as this may detect the oxygen consumption rate of each of the immune cells and/or the change in pH around the immune cell, and may be formed by using, for example, the optical means, and the oxygen sensor and/or the pH sensor immobilized in at least a part of the micro-well 101. Details thereof are the same as the contents described in "1. Information processing device 1", so that the description thereof is herein omitted.

### (3) Information processing device 1

Details of the information processing device 1 are the same as the contents described in "1. Information processing device 1", so that the description thereof is herein omitted. Note that, in the present technology, the detecting device 102 and the information processing device 1 may be connected to each other via a network.

### (4) Others

The cell analyzing system 10 according to the present technology may also be provided with other devices and the like such as a data accumulating device which accumulates the result of the analysis in addition to the above-described micro-well 101, detecting device 102, and information processing device 1 as long as the effects of the present technology are not impaired.

Note that the present technology may also take the following configuration.
(1) An information processing device at least including:
   an analyzing unit which analyzes a result of measurement over time of oxygen consumption rates of different types of immune cells collected from a subject stored for each cell in sealable micro-wells and/or a change in pH around the immune cell; and
   an output unit which outputs a prediction result of efficacy of an anticancer drug in the subject on the basis of a result of the analysis.
(2) The information processing device according to (1), in which the measured result is a result measured when the immune cell is stressed by drug stimulation and/or a change in external environment.
(3) The information processing device according to (1) or (2), in which the measured result is a result measured by using an oxygen sensor and/or a pH sensor immobilized in at least a part of the micro-well.
(4) The information processing device according to (3),
   in which the micro-well is formed by using a translucent material, and
   the measured result is a result of measurement over time of a change of the oxygen sensor and/or a change of the pH sensor by using an optical means with respect to the micro-well.
(5) The information processing device according to (4), in which the analyzing unit further analyzes a decay time of phosphorescence or fluorescence obtained from the oxygen sensor and/or the pH sensor.
(6) The information processing device according to any one of (1) to (5), in which the analyzing unit further performs profiling of energy metabolism of each immune cell on the basis of the measured result.
(7) The information processing device according to (6), in which the profiling is performed by analyzing a subtype of the immune cell determined from analysis of a surface antigen of the immune cell and a combination of energy metabolism of the immune cell of each subtype.
(8) The information processing device according to any one of (1) to (7), in which the immune cell includes any one selected from a group of CD8 positive T cells, CD4 positive helper T cells, and CD19 positive B cells.
(9) The information processing device according to any one of (1) to (8), in which an oligonucleotide for mRNA capturing is arranged in advance in at least a part of the micro-well.
(10) The information processing device according to (9), in which the oligonucleotide is an identification sequence different for a part or all of the micro-wells.
(11) The information processing device according to (1), in which the anticancer drug is a molecular target drug.
(12) The information processing device according to (11), in which the molecular target drug is a drug containing an anti-PD-1 antibody as an active ingredient.
(13) An information processing method at least performing:
   an analyzing procedure of analyzing a result of measurement over time of oxygen consumption rates of different types of immune cells collected from a subject stored for each cell in sealable micro-wells and/or a change in pH around the immune cell; and
   an output procedure of outputting a prediction result of efficacy of an anticancer drug in the subject on the basis of a result of the analysis.
(14) A cell analyzing system at least including:
   micro-wells which sealably store different types of immune cells collected from a subject;
   a detecting device which detects an oxygen consumption rate of each of the immune cells and/or a change in pH around the immune cell; and
   an information processing device which analyzes the oxygen consumption rate of the immune cell and/or the change in pH around the immune cell and outputs a prediction result of efficacy of an anticancer drug in the subject on the basis of a result of the analysis.

### REFERENCE SIGNS LIST

- 1: Information processing device
- 11: Analyzing unit
- 12: Output unit
- 10: Cell analyzing system
- 101: Micro-well
- 102: Detecting device

## Claims

1. An information processing device at least comprising:
an analyzing unit which analyzes a result of measurement over time of oxygen consumption rates of different types of immune cells collected from a subject stored for each cell in sealable micro-wells and/or a change in pH around the immune cell; and
an output unit which outputs a prediction result of efficacy of an anticancer drug in the subject on a basis of a result of the analysis.

2. The information processing device according to claim 1, wherein the measured result is a result measured when the immune cell is stressed by drug stimulation and/or a change in external environment.

3. The information processing device according to claim 1, wherein the measured result is a result measured by using an oxygen sensor and/or a pH sensor immobilized in at least a part of the micro-well.

4. The information processing device according to claim 3,
wherein the micro-well is formed by using a translucent material, and
the measured result is a result of measurement over time of a change of the oxygen sensor and/or a change of the pH sensor by using an optical means with respect to the micro-well.

5. The information processing device according to claim 4, wherein the analyzing unit further analyzes a decay time of phosphorescence or fluorescence obtained from the oxygen sensor and/or the pH sensor.

6. The information processing device according to claim 1, wherein the analyzing unit further performs profiling of energy metabolism of each immune cell on a basis of the measured result.

7. The information processing device according to claim 6, wherein the profiling is performed by analyzing a subtype of the immune cell determined from analysis of a surface antigen of the immune cell and a combination of energy metabolism of the immune cell of each subtype.

8. The information processing device according to claim 1, wherein the immune cell includes any one selected from a group of CD8 positive T cells, CD4 positive helper T cells, and CD19 positive B cells.

9. The information processing device according to claim 1, wherein an oligonucleotide for mRNA capturing is arranged in advance in at least a part of the micro-well.

10. The information processing device according to claim 9, wherein the oligonucleotide is an identification sequence different for a part or all of the micro-wells.

11. The information processing device according to claim 1, wherein the anticancer drug is a molecular target drug.

12. The information processing device according to claim 11, wherein the molecular target drug is a drug containing an anti-PD-1 antibody as an active ingredient.

13. An information processing method at least performing:
an analyzing procedure of analyzing a result of measurement over time of oxygen consumption rates of different types of immune cells collected from a subject stored for each cell in sealable micro-wells and/or a change in pH around the immune cell; and
an output procedure of outputting a prediction result of efficacy of an anticancer drug in the subject on a basis of a result of the analysis.

14. A cell analyzing system at least comprising:
micro-wells which sealably store different types of immune cells collected from a subject;
a detecting device which detects an oxygen consumption rate of each of the immune cells and/or a change in pH around the immune cell; and
an information processing device which analyzes the oxygen consumption rate of the immune cell and/or the change in pH around the immune cell and outputs a prediction result of efficacy of an anticancer drug in the subject on a basis of a result of the analysis.
